# EUROPÄISCHE PATENTSCHRIFT

(11) **EP 1 633 279 B1**
(45) Veröffentlichungstag und Bekanntmachung des Hinweises auf die Patenterteilung: **23.02.2011**
(21) Anmeldenummer: 04739803.7
(22) Anmeldetag: 11.06.2004
(51) Int. Cl.: A61F 2/04, A61M 27/00, A61F 2/06, A61M 29/02

(54) **SAUGSTENT**
SUCTION STENT
STENT D'ASPIRATION

(30) Priorität: 13.06.2003 DE 10327231
(43) Veröffentlichungstag der Anmeldung: 15.03.2006
(73) Patentinhaber: MNet GmbH Medizinische Datensysteme, 80333 München (DE)
(72) Erfinder: BENZ, Stefan, 72202 Nagold (DE); PFEFFER, Frank, 5200 Oslo (NO)
(74) Vertreter: Graf von Stosch, Andreas
(86) Internationale Anmeldenummer: PCT/EP2004/006311
(87) Internationale Veröffentlichungsnummer: WO 2004/110311

(56) Entgegenhaltungen:
- WO-A-01/21108
- US-A- 5 785 679
- US-A1- 2001 029 349

## Beschreibung

Die vorliegende Erfindung betrifft einen Stent zur Einführung in ein Hohlorgan, insbesondere den Gastrointestinaltrakt, eines menschlichen oder tierischen Patienten, der eine Vakuumversiegelung von Undichtigkeiten, wie Anastomoseinsuffizienzen, in dem Hohlorgan bewirkt.

Undichtigkeiten von chirurgischen Nahtverbindungen (Anastomosen) im Magen-Darmtrakt stellen die gefährlichste und damit wichtigste Komplikation nach Operationen im Bauchraum dar. Im Falle einer Undichtigkeit kommt es zum Austritt von Magen- bzw. Darminhalt in die Bauchhöhle und damit zu einer Bauchfellentzündung, die auch heute noch in etwa 20% der Fälle tödlich endet. Die Behandlung einer solchen Undichtigkeit ist abhängig von der genauen Lokalisation und der bereits eingetretenen Schädigung durch den ausgetretenen Darminhalt. Im besten Fall ist die Abheilung der Nahtverbindung verzögert und das funktionelle Ergebnis der Operation, d.h. beispielsweise die Kontinenz, beeinträchtigt. Um das Leben des Patienten nicht zu gefährden, sind häufig aber erheblich invasivere Maßnahmen wie eine Reoperation mit Aufhebung der Darmkontinuität und Anlage eines künstlichen Darmausgangs erforderlich. Dabei kann der künstliche Darmausgang nur in einem Teil der Fälle wieder zurückverlegt werden.

In Fällen, bei denen eine Aufhebung der Kontinuität des Magen-Darmtrakts nicht möglich ist, ist bisher versucht worden, Anastomoseinsuffizienzen mit einem endoskopisch platzierten endoluminalen, gecoverten Stent, insbesondere aus Metall (selbstexpandible Metallstents mit Kunststoffummantelung) abzudichten. Diese sind ursprünglich für die Behandlung von stenosierenden Tumoren in der Speiseröhre entwickelt worden und im Handel bspw. von Boston Scientific und Microvasive (Bezeichnung z.B. Ultraflex, gecovert) erhältlich.

Eine ausreichende Abdichtung der Nahtstellen in derartigen Hohlorganen gelingt jedoch mit den bisher verfügbaren Systemen nur bei einem kleinen Teil der Fälle, so dass sich dieses Vorgehen nicht als Standard durchsetzen konnte.

In der DE-A-199 49 334 wird bspw. ein Dickdarm-Stent beschrieben, der zur Implantation in ein Hohlorgan des menschlichen Körpers, insbesondere in den Darm, vorgesehen ist. Der in dieser Druckschrift offenbarte Stent weist bei ansonsten konventioneller Auslegung Mittel zum lösbaren Fixieren des Stents im Inneren des Hohlorgans, insbesondere des Darms, auf, um ein spontanes Abgehen des Stents im Gastrointestinaltrakt zu verhindern.

Die US 5,785,679 beschreibt eine Stent-Anordnung zur Einsetzung in einem von einem Aneurysma befallenen Blutgefäß. Diese Stent-Anordnung weist eine aufblasbare Hülle auf, welche eine Druckkammer bildet und durch einen Überdruck in der Kammer die Anordnung an ihrer Stelle im Blutgefäß hält, um das Aneurysma zu überbrücken.

Die im Stand der Technik bekannten Stents weisen jedoch besonders bei der Anwendung im Gastrointestinaltrakt spezifische Nachteile auf.

So erreichen die bekannten Stents meist keine ausreichende Abdichtung des Defekts, bspw. einer chirurgischen Nahtverbindung. Dies ist in der Regel auf die Inkongruenz des applizierten Stents mit der unregelmässig geformten Darmwand zurückzuführen. Des weiteren können im Bereich von undichten Nahtverbindungen keine selbstexpandierbaren Stents mit hohen Rückstellkräften eingesetzt werden, um eine vollständige Abdichtung zu erreichen, da dies zu einer weiteren Schädigung oder gar Sprengung der Nahtverbindung führen könnte.

Wird in Ausnahmefällen tatsächlich eine vollständige Abdichtung des Defekts erreicht, kann der aus dem Hohlorgan ausgetretene Inhalt, insbesondere der Darminhalt, nicht abfließen.

Daher kommt es besonders im Magen-Darmtrakt fast zwangsläufig zu einer Abszessbildung an der Nahtverbindung und damit zu einer weiteren Gefährdung der Lokalsituation und des Patienten.

Der vorliegenden Erfindung liegt daher die Aufgabe zugrunde, einen Stent bereitzustellen, der in Hohlorganen des menschlichen oder tierischen Körpers auftretende lokale Defekte, bspw. undichte chirurgische Nahtverbindungen, wirksam abdichtet und gleichzeitig etwaige Flüssigkeitsansammlungen an derartigen Defekten wirksam abführt.

Diese Aufgabe wird durch den in den Ansprüchen gekennzeichneten Stent der vorliegenden Erfindung gelöst.

Insbesondere wird erfindungsgemäß ein Stent zur Einführung in ein Hohlorgan des menschlichen oder tierischen Körpers, insbesondere den Gastrointestinaltrakt, vor allem in die Speiseröhre oder den Darm, bspw. in Rectum, Sigma, Colon descendens oder Colon transversum, bereitgestellt, umfassend
- einen radial expandierbaren und in Längsrichtung offenen rohrartigen Hohlkörper,
- ein den Hohlkörper mindestens bereichsweise radial umhüllendes poröses formbares Material und
- eine in das vorstehend genannte poröse formbare Material eingebrachte Drainage, welche zum Anlegen eines Unterdrucks ausgelegt ist,
wobei das Lumen des Hohlkörpers gegenüber dem porösen formbaren Material luft- und wasserdicht ist.

Somit wird erfindungsgemäß das vorstehende Problem der möglichst vollständigen Abdichtung von Defekten in Hohlorganen und die gleichzeitige Möglichkeit der Entfernung von aus dem Hohlorgan ausgetretener Flüssigkeit wie bspw. dem Darminhalt dadurch gelöst, daß der Defekt, bspw. ausgehend von einer Anastomoseinsuffizienz, vakuumversiegelt wird.

Der radial expandierbare und in Längsrichtung offene rohrartige Hohlkörper des erfindungsgemäßen Stents kann bspw. ein selbstexpandibler bzw. selbstexpandierbarer Metall- oder Kunststoffstent sein, wie er im z.B. in US 5,876,448 beschrieben ist. Der diesbezügliche Offenbarungsgehalt dieser Druckschrift ist ausdrücklich Bestandteil der vorliegenden Offenbarung. Ein Stent im Sinne der vorliegenden Erfindung ist ein expandierbarer Hohlkörper, wobei Expansion jeder Art von Volumenzunahme bedeutet, insbesondere durch Aufblasen des Hohlkörpers bzw. durch mechanische Erweiterung (ggf. Aufweitung) des Hohlkörpers. Ein derartiger Stent wird in Körperhohlräume eingesetzt (bspw. mit tubusartiger Struktur), wobei unter einem Stent beispielsweise auch ein Ballon verstanden werden kann, wobei dieser wiederum typischerweise durch Aufblasen expandiert wird. Der Stellt ist mindestens bereichsweise, vorzugsweise im wesentlichen vollständig radial mit dem porösen formbaren Material ummantelt. Das poröse formbare Material kann geschlossenporig, d.h. in Form eines Schaums, oder offenporig, d.h. schwammartig, ausgestaltet sein. Bevorzugte Materialien hierfür sind Kunststoffschäume, bspw. solche die aus Polyurethanen, Polyvinylalkoholen bzw. Gemischen solcher Kunststoffe bestehen bzw. diese umfassen. Somit stellt mindestens ein Teil des erfindungsgemäßen Stents bspw. einen selbstexpandierbaren, ansonsten in üblicher Weise ausgelegten Metall- oder Kunststoffstent mit Kunststoffummantelung dar.

Der luminale Durchmesser des erfindungsgemäßen Stents, d.h. des radial expandierbaren Hohlkörpers, liegt bspw. im Bereich von etwa 10 bis 50 mm, vorzugsweise 15. bis 35 mm, insbesondere 15 bis 30 mm; ganz besonders bevorzugt beträgt er etwa 25 mm (z.B. bei Anwendungen im Dickdarmbereich) oder etwa 18 mm (bspw. zum Einsatz in der Speiseröhre). In jedem Fall wird der Durchmesser des Stents derart gewählt, daß je nach Anwendungsgebiet der Durchgang entsprechender Stoffe durch das jeweilige Hohlorgan, im Fall des Darmtrakts die intestinale Nahrungspassage, nicht behindert wird.

Erfindungsgemäß ist das formbare poröse Material, bspw. ein Schaumstoff, gegen das Lumen luft- und wasserdicht. Gemäß einer bevorzugten Ausführungsform erfolgt, sofern der Hohlkörper nicht primär dicht ist, die Abdichtung mindestens bereichsweise, insbesondere vollständig, durch eine luft- und wasserundurchlässige Folie. Besonders vorteilhaft für diesen Zweck sind Kunststofffolien, die bspw. ein Polyurethan, Latex und/oder Silikon enthalten oder daraus bestehen.

Die Folie kann dabei als Wicklung um den Hohlkörper vorliegen, wobei Ansatzstellen selbstverständlich abgedichtet sein müssen, was bspw. durch eine Verschweißung im Falle einer Kunststofffolie leicht zu bewerkstelligen ist. Bevorzugt sind jedoch erfindungsgemäße Folien, insbesondere Kunststofffolien, die schlauchförmig ausgebildet sind. Vorzugsweise überragt die Folie den Körper dabei mindestens an einem Ende, mehr bevorzugt überragt sie den Hohlkörper an beiden Enden. Weiterhin ist es erfindungsgemäß bevorzugt, wenn die Folie, bspw. eine schlauchförmige Kunststofffolie, den Hohlkörper an beiden Enden überragt und der Durchmesser dabei nach peripher weiter zunimmt.

Der Durchmesser des Hohlkörpers nimmt gemäß einer weiteren bevorzugten Ausführungsform der vorliegenden Erfindung in einem oder beiden Endbereich(en) zu. Bevorzugt ist es, wenn der Durchmesser an beiden Endbereichen zunimmt. In diesem Fall nimmt die Dicke des porösen formbaren Materials, bspw. des Schaumstoffs, in dem bzw. den Endbereich(en) des Hohlkörpers ab, wobei es besonders bevorzugt ist, wenn ihre Dicke im gleichen Maß abnimmt wie der Durchmesser des Hohlkörpers in dem bzw. den Endbereich(en) zunimmt. Das poröse formbare Material, z.B. ein Kunststoffschaum, liegt dabei bspw. in einer Dicke von etwa 5 bis etwa 10 mm vor.

Der erfindungsgemäße Stent umfaßt weiter eine in das poröse formbare Material eingebrachte Drainage, die zum Anlegen eines Unterdrucks ausgelegt ist (Saugdrainage). Gemäß einer bevorzugten Ausführungsform ist die Saugdrainage bspw. von luminal durch eine Durchtrittsstelle in der erfindungsgemäßen Folie in das formbare poröse Material eingebracht. Dabei sollte gewährleistet sein, daß die Durchtrittsstelle in der Folie gegen die Drainage, die bspw. aus einem entsprechenden Kunststoffschlauch, wie einem Fachmann bekannt ist, ausgebildet ist, abgedichtet ist. Bevorzugte Ausführungsformen derartiger Abdichtungen sind bspw. in DE-A-44 33 450, auf deren diesbezüglichen Offenbarungsgehalt vollumfänglich Bezug genommen wird, offenbart. Gemäß einer besonderen Ausfühmngsform erfolgt die Dichtung an der Durchtrittsstelle durch ein leicht verformbares, nicht fließendes Dichtungsmaterial. Dieses Dichtungsmaterial ist vorzugsweise ein Silikon, ein Hydrokolloid, ein Lyogel, insbesondere ein Hydrogel, und läßt sich somit leicht von Hand verformen und gegebenenfalls vorliegenden Unebenheiten, die an der Durchtrittsstelle beim Drainageschlauch oder eventuellen Falten der Folie vorliegen, anpassen, wobei das Dichtungsmaterial in die vorhandenen Undichtigkeiten eindringt und diese verschließt. Die Verformbarkeit und die Fließeigenschaften des Dichtungsmaterials werden vorzugsweise derart eingestellt, daß eine leichte Verformbarkeit und ein gutes Eindringen in die Undichtigkeiten gewährleistet ist, jedoch ein Abfließen des Dichtungsmaterials unter der Wirkung des an die Drainage angelegten Druckgradienten ausgeschlossen ist.

Die gesamte Konstruktion des erfindungsgemäßen Stents ist vorzugsweise vollständig expandierbar und kann gemäß üblichen Applikationsmaßnahmen an den Applikationsort im Organ, Insbesondere dem Gastrointestinaltrakt, vorzugsweise in die Speiseröhre, den Darm, vor allem in Rectum, Sigma, Colon descendens oder Colon transversum, gebracht werden. Nachfolgend wird die Erfindung in Bezug auf die beigefügten Figuren anhand einer beispielhaften Ausführungsform des erfindungsgemäßen Stents beschrieben.
- Fig. 1: zeigt einen Längsschnitt durch einen erfindungsgemäß applizierten Stent im Darm, der eine Anastomoseinsuffizienz aufweist.
- Fig. 2: zeigt in schematischer Darstellung einen Längsschnitt durch einen erfindungsgemäßen Stent.
- Fig. 3: zeigt in einer weiteren schematischen Darstellung einen Querschnitt durch den in der Fig. 2 gezeigten Stent.

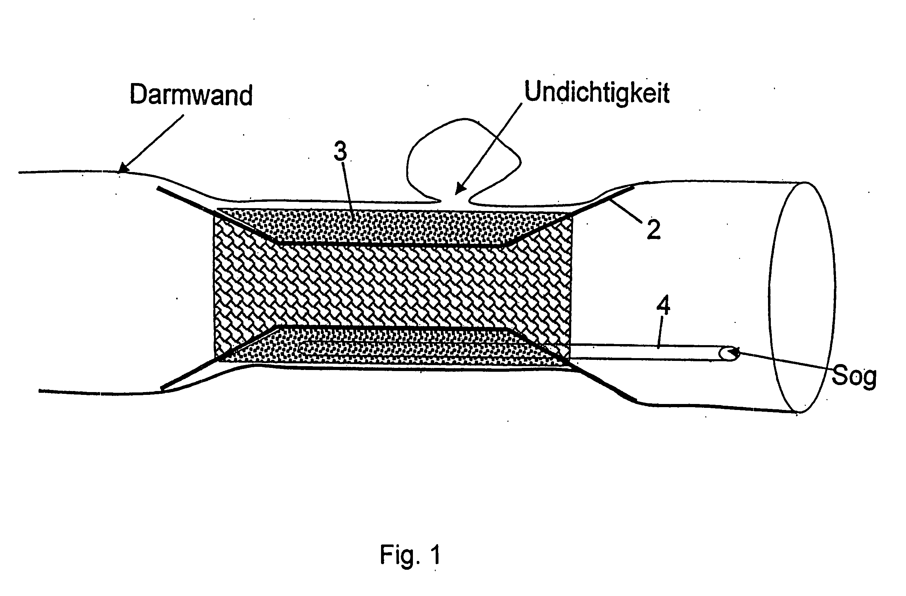

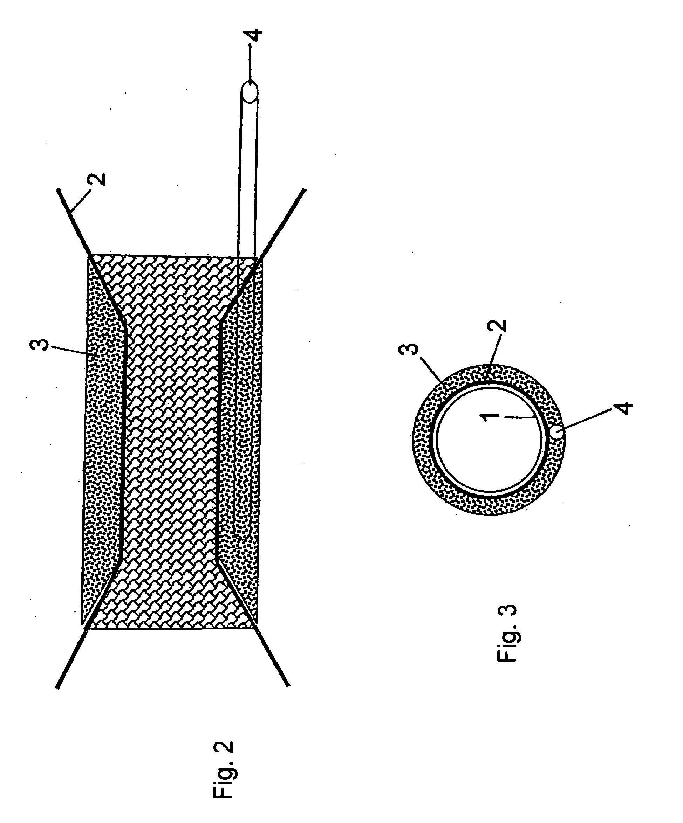

Bezugnehmend auf die Fig. 1 bis 3, wird erfindungsgemäß ein endoskopisch platzierbarer selbstexpandierbarer Stent (Metall oder Kunststoff) (1) mit einem weichen Kunststoffschaum (3) zirkulär umkleidet. Der Schaumstoff ist gegen das Lumen mit einer luftdichten und wasserdichten Kunststofffolie (2) abgedichtet. Von luminal ist in den Schaum (3) eine Saugdrainage (4) durch diese Kunststofffolie (2) eingebracht, wobei die Folie (2) gegen die Drainage (4) an der Durchtrittsstelle abgedichtet ist. Nach beiden Enden vergrößert sich der Durchmesser des Stents (1), wohingegen die Dicke des Schaumes (3) abnimmt. Die Dichtungsfolie (2) überragt den Stent (1) zirkulär, wobei der Durchmesser nach peripher weiter zunimmt. Üblicherweise ist die gesamte Konstruktion expandierbar und wird über einen drahtgeführten Applikator unter endoskopischer- oder Röntgenkontrolle an den Applikationsort gebracht, vorliegend in den Darm eingeführt, der an der Applikationsstelle eine Undichtigkeit, bspw. eine Anastomoseinsuffizienz, aufweist. Wie in Fig. 1 gezeigt, wird der Stent vorzugsweise über einen endoskopisch gelegten Führungsdraht derart platziert, daß die Undichtigkeit in der Mitte des Stents zu liegen kommt. Nach Freisetzen des Stents (Selbstexpansion) expandiert dieser auf seine vorgegebene Größe und liegt dann der Darmwand, wie in Fig. 1 gezeigt, locker an. Die am Stent vorgesehene Saugdrainage (4) wird je nach Einsatzort des Stents peranal, peroral oder pemasal ausgeleitet, und es wird ein Sog angelegt. Die Darmwand wird dadurch zirkulär an den Stent angesaugt, so daß dieser die Undichtigkeit vollständig abdeckt und ein Austritt von Flüssigkeit aus dem Hohlorgan, vorliegend dem Darm, verhindert wird. Gleichzeitig wird etwaig vorliegende Flüssigkeit, die sich im Bereich der Undichtigkeit jenseits der Darmwand angesammelt hat, an- und durch die Saugdrainage abgesaugt. Der Stent kann nunmehr solange *in situ* verbleiben, bis sich die Situation des Patienten stabilisiert hat. Danach kann eine Extraktion des Stents erfolgen.

Es wird daher unter einem weiteren Gesichtspunkt der vorliegenden Erfindung ein Verfahren zum Abdichten von in einem Hohlorgan des menschlichen oder tierischen Körpers vorliegenden Undichtigkeiten, insbesondere im Magen-Darmtrakt, bspw. den vorstehend genannten Teilen des Darms, bereitgestellt, umfassend die Schritte:
(a) Einbringen des erfindungsgemäßen Stents in das Hohlorgan, so daß die Undichtigkeit des Hohlorgans an der Längsseite des Stents, vorzugsweise im Bereich der Mitte der Längsseite, insbesondere genau in der Mitte, liegt,
(b) Freisetzen des Stents, so daß sich dieser in die vorgegebene Größe expandiert und
(c) Anlegen eines Unterdrucks (Sogs) an die Drainage des Stents.

Somit wird erfindungsgemäß erstmalig ein intraluminaler Stent als Träger einer Vakuumversiegelung für Undichtigkeitsstellen in Hohlorganen und ein entsprechendes Verfahren zum Abdichten der Undichtigkeiten bereitgestellt. Mit diesem Verfahren ist es insbesondere möglich, durch einen intraluminalen Eingriff eine Anastomoseinsuffizienz, insbesondere im Dick- und Enddarmbereich, sicher abzudichten. Der erfindungsgemäße Stent begünstigt somit das Management von Patienten mit kritischen Anastomoseverhältnissen in besonders positiver Weise. Dabei führt die Anwendung des erfindungsgemäßen Stents zu einem Überleben des Patienten in einer ansonsten vital bedrohlichen Situation. Unter Verwendung des erfindungsgemäßen Stents wird der Patient etwa 5 Tage bis 1 Woche Bettruhe (um eine sichere Abdichtung zu gewährleisten) einhalten. Ohne die vorliegende Erfindung wäre in derartigen Situationen in der Regel eine Operation und- im Fall von Anastomoseinsuffizienzen im Darmbereich - häufig die Anlage eines künstlichen Darmausgangs erforderlich, der nicht in allen Fällen und zum Teil nur mit erheblicher Morbidität zurückverlegt werden kann.

## Patentansprüche

1. Stent zur Einführung in ein Hohlorgan des menschlichen oder tierischen Körpers, vorzugsweise in den Gastrointestinaltrakt, insbesondere den Darm, umfassend
- einen radial expandierbaren und in Längsrichtung offenen rohrartigen Hohlkörper (1),
und **gekennzeichnet durch**
- ein den Hohlkörper (1) mindestens bereichsweise radial umhüllendes poröses formbares Material (3) und
- eine in das Material (3) eingebrachte Drainage (4), die zum Anlegen eines Unterdrucks ausgelegt ist,
wobei das Lumen des Hohlkörpers (1) gegenüber dem Material (3) luft- und wasserdicht ist.

2. Stent nach Anspruch 1, wobei der Hohlkörper (1) gegen das Material (3) mindestens bereichsweise durch eine luft- und wasserundurchlässige Folie (2) abgedichtet ist.

3. Stent nach Anspruch 2, wobei die Folie (2) schlauchförmig ausgebildet ist.

4. Stent nach Anspruch 2 oder 3, wobei die Folie (2) den Hohlkörper (1) an mindestens einem Ende, vorzugsweise beiden Enden überragt.

5. Stent nach Anspruch 4, wobei die Folie den Stent an beiden Enden überragt und der Durchmesser der Folie (2) in den den Hohlkörper (1) überragenden Bereichen zunimmt.

6. Stent nach einem der Ansprüche 2 bis 5, wobei die Drainage (4) durch eine Durchtrittsstelle in der Folie (2) in das Material (3) eingebracht ist.

7. Stent nach Anspruch 6, wobei die Folie (2) an der Durchtrittsstelle gegen die Drainage (4) durch ein leicht verformbares, nicht fließendes Dichtungsmaterial abgedichtet ist.

8. Stent nach Anspruch 7, wobei das Dichtungsmaterial ein Silikon, Hydrokolloid oder Lyogel, insbesondere ein Hydrogel ist.

9. Stent nach einem der Ansprüche 1 bis 8, wobei der Durchmesser des Hohlkörpers (1) in einem oder beiden Endbereich(en) zunimmt.

10. Stent nach Anspruch 9, wobei die Dicke des Materials (3) in dem bzw. den Endbereich(en) des Hohlkörpers abnimmt.

11. Stent nach Anspruch 10, wobei die Dicke des Materials (3) im gleichen Maß abnimmt wie der Durchmesser des Hohlkörpers (1) in dem bzw. den Endbereich(en) zunimmt.

12. Stent nach einem der Ansprüche 1 bis 11, wobei das Material (3) ein Kunststoffschaum ist.

13. Stent nach Anspruch 12, wobei der Kunststoffschaum aus der Gruppe, bestehend aus Polyurethanen und Polyvinylalkoholen, ausgewählt ist.

14. Stent nach einem der Ansprüche 2 bis 13, wobei die Folie (2) einen Kunststoff enthält oder daraus besteht.

15. Stent nach Anspruch 14, wobei der Kunststoff aus der Gruppe, bestehend aus Polyurethanen, Latex und Silikon, ausgewählt ist.

## Claims

1. Stent for introduction into a hollow organ of the human or animal body, preferably into the gastrointestinal tract, in particular the intestine, comprising:
a radially expandable tubular hollow body (1) open in the longitudinal direction, and **characterized by**
a porous shapeable material (3) radially sheathing the hollow body (1) at least in certain regions, and
a drainage means (4) introduced into the material (3), which is configured to apply a subnormal pressure,
wherein the lumen of the hollow body (1) is air- and water-tight with respect to the material (3).

2. Stent according to claim 1, wherein the hollow body (1) being sealed from the material (3), at least in certain regions, by an air- and water-tight film (2).

3. Stent according to claim 2, wherein the film (2) is tubular.

4. Stent according to claim 2 or 3, wherein the film (2) projects beyond the hollow body (1) at least at one end, preferably at both ends.

5. Stent according to claim 4, wherein the film projects beyond the stent at both ends and the diameter of the film (2) increases in the regions projecting beyond the hollow body (1).

6. Stent according to any one of claims 2 to 5, wherein the drainage means (4) is introduced into the material (3) via a point of passage in the film (2).

7. Stent according to claim 6, wherein, at the point of passage, the film (2) is sealed from the drainage means (4) by an easily deformable, non-flowing sealant.

8. Stent according to claim 7, wherein the sealant is a silicone, hydrocolloid or lyogel, in particular a hydrogel.

9. Stent according to any one of claim 1 to 8, wherein the diameter of the hollow body (1) increases in one or both end region(s).

10. Stent according to claim 9, wherein the thickness of the material (3) decreases in the end region(s) of the hollow body.

11. Stent according to claim 10, wherein the thickness of the material (3) decreases to the same extent as the diameter of the hollow body (1) increases in the end region(s).

12. Stent according to any one of claims 1 to 11, wherein the material (3) is a plastics material foam.

13. Stent according to claim 12, wherein the plastics material foam is selected from the group consisting of polyurethanes and polyvinyl alcohols.

14. Stent according to any one of claims 2 to 13, wherein the film (2) contains a plastics material or consists thereof.

15. Stent according to claim 14, wherein the plastics material is selected from the group consisting of polyurethanes, latex and silicone.

## Revendications

1. Stent pour l'introduction dans un organe creux du corps humain ou animal, de préférence par ingestion, en particulier dans l'intestin, comprenant
- un corps creux (1) à la manière d'un tube, expansible radialement et ouvert en direction longitudinale,
et **caractérisé par**
- un matériau (3) déformable poreux, enveloppant radialement au moins par tronçons le corps creux (1) et
- un drainage (4) placé dans le matériau (3), réalisé pour produire une dépression, sachant que le lumen du corps creux (1) est étanche à l'air et à l'eau par rapport au matériau (3).

2. Stent selon la revendication 1, sachant que le corps creux (1) est étanche par rapport au matériau (3) au moins par tronçons grâce à un film imperméable à l'air et à l'eau (2).

3. Stent selon la revendication 2, sachant que le film (2) est réalisé sous forme de tuyau flexible.

4. Stent d'aspiration selon la revendication 2 ou 3, sachant que le film (2) dépasse du corps creux (1) au niveau au moins d'une extrémité, de préférence au niveau des deux extrémités.

5. Stent selon la revendication 4, sachant que le film dépasse le stent au niveau des deux extrémités et que le diamètre du film (2) augmente dans les zones dépassant le corps creux (1).

6. Stent selon l'une quelconque des revendications 2 à 5, sachant que le drainage (4) est introduit dans le matériau (3) grâce à un point de passage dans le film (2).

7. Stent selon la revendication 6, sachant que le film (2) est étanche au niveau du point de passage par rapport au drainage (4) grâce à un matériau d'étanchéité légèrement déformable, non liquide.

8. Stent selon la revendication 7, sachant que le matériau d'étanchéité est un silicone, un hydrocolloïde ou un lyogel, en particulier un hydrogel.

9. Stent selon l'une quelconque des revendications 1 à 8, sachant que le diamètre du corps creux (1) augmente dans l'une ou dans les deux zones d'extrémité.

10. Stent selon la revendication 9, sachant que l'épaisseur du matériau (3) diminue dans la ou les deux zones d'extrémité.

11. Stent selon la revendication 10, sachant que l'épaisseur du matériau (3) diminue dans la même proportion que le diamètre du corps creux (1) dans la ou les deux zones d'extrémité.

12. Stent selon l'une quelconque des revendications 1 à 11, sachant que le matériau (3) est une mousse en plastique.

13. Stent selon la revendication 12, sachant que la mousse en plastique est choisie parmi le groupe constitué de polyuréthanes et d'alcools polyvinyliques.

14. Stent selon l'une quelconque des revendications 2 à 13, sachant que le film (2) contient une matière plastique ou en est composé.

15. Stent selon la revendication 14, sachant que la matière plastique est choisie parmi le groupe constitué de polyuréthanes, de latex et de silicone.
